# FASCICULE DE BREVET EUROPEEN

(11) **EP 2 861 672 B1**
(45) Date de publication et mention de la délivrance du brevet: **02.01.2019**
(21) Numéro de dépôt: 13733352.2
(22) Date de dépôt: 06.06.2013
(51) Int. Cl.: C09B 67/00, A61K 8/58, A61Q 19/00, A61K 8/02, C09B 67/08

(54) **PIGMENT ORGANIQUE ENROBÉ ET COMPOSITION COSMÉTIQUE**
BESCHICHTETES ORGANISCHES PIGMENT UND KOSMETISCHE ZUSAMMENSETZUNG
COATED ORGANIC PIGMENT AND COSMETIC COMPOSITION

(30) Priorité: 15.06.2012 FR 1255598; 05.07.2012 US 201261668249 P
(43) Date de publication de la demande: 22.04.2015
(73) Titulaire: L'OREAL, 75008 Paris (FR)
(72) Inventeur: JEANNE-ROSE, Valérie, F-95100 Argenteuil (FR)
(74) Mandataire: Kromer, Christophe
(86) Numéro de dépôt international: PCT/FR2013/051286
(87) Numéro de publication internationale: WO 2013/186462

(56) Documents cités:
- FR-A1- 2 795 949
- JP-A- 2001 011 342
- US-A1- 2002 069 790
- US-A1- 2007 238 257
- US-A1- 2008 199 523

## Description

La présente invention a pour objet un procédé de préparation d'un pigment organique enrobé, un pigment organique enrobé obtenu selon ce procédé et une composition cosmétique comprenant un tel pigment organique enrobé. L'invention a également pour objet un procédé de maquillage des matières kératiniques utilisant ladite composition. La composition et le procédé de maquillage conviennent pour les matières kératiniques comme la peau, les lèvres, les cheveux, les cils, les sourcils et les ongles d'être humain. La composition se présente notamment sous forme de produit de maquillage, en particulier de vernis à ongles, de produit pour les lèvres, de produit de maquillage du corps, de fond de teint, de fard à paupières ou à joues, d'eye-liner, de produit anti-cernes, de mascara.

Les compositions de maquillage contiennent généralement des matières colorantes pour conférer à la composition la couleur souhaitée. Les matières colorantes peuvent être des pigments minéraux ou organiques. Il est connu que les colorants fluorescents permettent d'obtenir des couleurs vives et très luminueuses visibles en particulier sous un éclairage ultra-violet.
Un composé fluorescent est un composé capable d'absorber dans le rayonnement UV ou visible à une longueur d'onde λ_{abs} comprise entre 250 et 800 nm et capable de réémettre dans le domaine du visible à une longueur d'onde d'émission λ_{ém} comprise entre 400 et 800 nm.
En cosmétique, peu de colorants fluorescents sont autorisés en maquillage (on peut citer les Red 21, Red 22, Red 27, Red 28, Ornage 5, Yellow 11) ce qui limite la variété et l'accesssibilité à ces types de pigments pour pouvoir formuler des produits de maquillage dans des teintes variées tout en présentant des effets de fluorescence particulièrement attrayants. Il est connu les pigments organiques en-trappés dans une matrice polyester comme les produits vendus par la société Dayglo sous les références Dermaglo , notamment Dermaglo DG-R222, DG-R228, DG-R422, DG-R428, DG-O205.

Par ailleurs, il est connu de l'état de la technique d'enrober les pigments organiques avec des revêtements siliciés.
US6355260 décrit des pigments organiques ou minéraux dans la surface est revêtue avec un composé minéral tel qu'un polymère d'acide silicique ou un dépôt obtenue après hydrolyse de tétraéthoxysilane ou bien encore un dépôt d'alumine. L'hydrolyse de tétraéthoxysilane ne permet pas de révéler la fluorescence du pigement organique.
EP-A-581651 décrit des particules colorées sphériques comprenant un pigment organique ou minéral revêtu d'un composé métallique hydraté obtenu par hydrolyse d'un composé métallique alcoxy. Dans les exemples 1, 4, 6 et 7, des pigments sont traités par hydrolyse de tétraéthoxysilane.
JP-A-2002-308716 décrit un pigment organique Red 202 enrobé par hydrolyse de tétraéthoxysilane.
JP-A-2002-309173 décrit des pigments organiques et minéraux revêtues de silice obtenue par hydrolyse de tétraéthoxysilane.
JP-A-2001-011342 décrit des pigments traités par hydrolyse de tétraéthoxysilane et d'alkylalkoxysilane.

FR-A-2795949 décrit à l'exemple 3-5 des particules formées à partir d'oxyde de zinc, de polyéthylènemalate, d'octadecyltriméthoxysilane, de diméthylpolysiloxane dont le mélange est malaxé, pulvérisé et traité dans un broyeur à jet

Un besoin existe pour disposer d'autres pigments organiques enrobés présentant de bonne propriétés de fluorescence pour permettre d'élargir la palette de pigments fluorescents disponibles pour la formulation des produits de maquillage.
Le demandeur a découvert, de façon surprenante, qu'il est possible d'exalter la fluorescence de pigments organiques en enrobant ces pigments selon un procédé utilisant un composé silicié particulier. Ce procédé permet de former un revêtement de silice alkylée qui confère au pigment organique ainsi enrobé de bonnes propriétés de fluorescence , permettant ainsi d'obtenir des produits de maquillage ayant des teintes vives et lumineuses. Le maquillage obtenu avec ces pigments présente également une bonne couvrance.

De façon plus précise, l'invention a pour objet un procédé de préparation d'un pigment organique enrobé comprenant les étapes suivantes :
a) on prépare un mélange anhydre
   (i) d'un pigment organique et (ii) d'un composé silicié de formule (I) :

      [R₁-O]₃Si-R₂ (I)

      dans laquelle R₁ désigne un radical alkyle en C₁-C₄, R₂ désigne un radical alkyle en C₁-C₈ ;
      en l'absence de tétraalkyl(C₁-C₄)orthosilicate ;
      et éventuellement (iii) d'une huile ;
b) ledit mélange est mis en contact avec une composition aqueuse comprenant un tensioactif choisi parmi les sels d'alkyl (C₈-C₁₆) sulfate et les alkyl(C₆-C₁₀)phénol oxyéthyléné comprenant de 5 à 40 motifs d'oxyde d'éthylène ;
c) récupération du pigment enrobé sous forme de poudre .

L'invention a aussi pour objet un pigment organique enrobé susceptible d'être obtenu selon le procédé tel que défini précédemment.
L'invention a également pour objet un pigment organique enrobé d'un revêtement de silice alkylée telle que définie ci-après.
Un autre objet de l'invention est une composition cosmétique, notamment une composition de maquillage, comprenant, dans un milieu cosmétiquement acceptable, au moins un pigment organique enrobé tel que défini précédemment.

L'invention a aussi pour objet un procédé de maquillage des matières kératiniques comprenant l'application sur lesdites matières kératiniques d'une composition cosmétique telle que définie précédemment.

Il est connu du document US2008/0199523 des particules comprenant un dopant hydrophobe destiné à être relargué. Le dopant peut être un colorant fluorescent tel que le solvent blue 35 dye et le sudan red dye. Ces colorants sont solubles dans les corps gras et ne correspondent pas à un pigment organique non soluble dans les huiles comme l'isododécane, la décamethyl cyclopentasiloxane, les benzoates d'alcool en C₁₂-C₁₅. Les particules sont préparées par encapsulation du dopant lors de la polymérisation sol-gel de 3-aminopropyltriethoxysilane et de vinyltrime-thoxysilane dans l'eau en présence de tensioactif PEG-9 nonyl phenyl ether (exemples 7 et 8). Ce document ne préconise par d 'effectuer l'encapsulation de pigment organiques non solubles dans les huiles pour améliorer leurs propriétés de fluorescence et d'obtenir des produits de maquillage ayant des teintes vives et lumineuses, ayant une bonne couvrance.

On entend par milieu cosmétiquement acceptable un milieu compatible avec les matières kératiniques d'être humain, telles que la peau, les lèvres, les cheveux, les cils, les ongles.

Par pigment organique on entend un pigment organique insoluble dans l'eau à 25 °C, notamment à une teneur de 1 % en poids et également insoluble dans les huiles choisies parmi l'isododécane, la décamethyl cyclopentasiloxane, les benzoates d'alcool en C₁₂-C₁₅ tel que celui vendu sous la dénomination « Finsolv TN » par la société INNOSPEC ACTIVE CHEMICALS, à 25 °C , notamment à une teneur à 1 % en poids.

Les pigments organiques destinés à être enrobés peuvent être par exemple :
- le carmin de cochenille,
- les pigments organiques de colorants azoïques, anthraquinoniques, indigoïdes, xanthéniques, pyréniques, quinoliniques, de triphénylméthane, de fluorane ;
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane. Ces colorants comportent généralement au moins un groupe acide carboxylique ou sulfonique.

La laque organique peut aussi être supportée par tout support compatible tel qu' un support minéral comme les particules d'alumine, d'argile, de zircone ou d'oxydes métalliques, notamment d'oxyde de zinc ou d'oxyde de titane, de talc, de carbonate de calium, de sulfate de barium. De préférence, le support minéral est choisi parmi l'alumine, l'oxyde de titane et le sulfate de barium. La laque organique peut également être supportée par un support tel que la colophane ou le benzoate d'aluminium.

Parmi les pigments organiques, on peut citer le D&C Red n°7.

Parmi les laques organiques, on peut en particulier citer celles connues sous les dénominations suivantes :
D & C Red n° 2 Aluminium lake
D & C Red n° 3 Aluminium lake
D & C Red n° 4 Aluminium lake
D & C Red n° 6 Aluminium lake
D & C Red n° 6 Barium lake
D & C Red n° 6 Barium/Strontium lake
D & C Red n° 6 Strontium lake
D & C Red n° 6 Potassium lake
D & C Red n° 7 Aluminium lake
D & C Red n° 7 Barium lake
D & C Red n° 7 Calcium lake
D & C Red n° 7 Calcium/strontium lake
D & C Red n° 7 Zirconium lake
D & C Red n° 8 Sodium lake
D & C Red n° 9 Aluminium lake
D & C Red n° 9 Barium lake
D & C Red n° 9 Barium/Strontium lake
D & C Red n° 9 Zirconium lake
D & C Red n° 10 Sodium lake
D & C Red n° 19 Aluminium lake
D & C Red n° 19 Barium lake
D & C Red n° 19 Zirconium lake
D & C Red n° 21 Aluminium lake
D & C Red n° 21 Zirconium lake
D & C Red n° 22 Aluminium lake
D & C Red n° 27 Aluminium lake
D & C Red n° 27 Aluminium/Titanium/Zirconium lake
D & C Red n° 27 Barium lake
D & C Red n° 27 Calcium lake
D & C Red n° 27 Zirconium lake
D & C Red n° 28 Aluminium lake
D & C Red n° 30 lake
D & C Red n° 31 Calcium lake
D & C Red n° 33 Aluminium lake
D & C Red n° 34 Calcium lake
D & C Red n° 36 lake
D & C Red n° 40 Aluminium lake
D & C Blue n° 1 Aluminium lake
D & C Green n° 3 Aluminium lake
D & C Orange n° 4 Aluminium lake
D & C Orange n° 5 Aluminium lake
D & C Orange n° 5 Zirconium lake
D & C Orange n° 10 Aluminium lake
D & C Orange n° 17 Barium lake
D & C Yellow n° 5 Aluminium lake
D & C Yellow n° 5 Zirconium lake
D & C Yellow n° 6 Aluminium lake
D & C Yellow n° 7 Zirconium lake
D & C Yellow n° 10 Aluminium Lake
FD & C Blue n° 1 Aluminium lake
FD & C Red n° 4 Aluminium lake
FD & C Red n° 40 Aluminium lake
FD & C Yellow n° 5 Aluminium lake
FD & C Yellow n° 6 Aluminium lake

Les composés chimiques correspondant à chacun des pigments organiques cités précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association ».

On peut également utiliser les laques de colorants naturels tels que les laques de curcumine, de riboflavine, d'azorubine, d'amarante, de chlorophylle, de caroténoïdes comme le lycopène, d'anthocyanes, de sorgho, d'acide carminique.

Le composé silicié de formule (I) décrit précédemment, utilisé dans le procédé selon l'invention, permet de former en surface du pigment organique un revêtement d'enrobage de type silice alkylée. Cet enrobage est obtenu par hydrolyse et condensation du composé silicié (I) qui se produit après contact avec l'eau.

Dans le composé silicé (I), R₁ désigne un radical alkyle en C₁-C₄ et R₂ désigne un radical alkyle en C₁-C₈ . De préférence, R₂ désigne un radical alkyle en C₁-C₄.

Pour le composé silicié (I) défini précédemment, avantageusement R₁ désigne un radical alkyle en C₂-C₃ et R₂ désigne un radical alkyle en C₁-C₄.

Préférentiellement, R₁ est un radical éthyle et R₂ est un radical méthyle.

Le composé silicié (I) peut être choisi parmi le méthyltriéthoxysilane, le propyltri-méthoxysilane, le méthyltriméthoxysilane.

De préférence, le composé silicié (I) est le méthyltriéthoxysilane.

Le composé silicié (I) forme, après hydrolyse et condensation, un réseau de silice alkylée présentant des unités répétitives de type (SiO_{3/2}R₂), R₂ désignant un groupe alkyle en C₁-C₈.

Ainsi, le pigment est enrobé d'un revêtement comprenant une silice alkylée présentant ces unités répétitives.

Le composé silicié (I) peut généralement être présent à raison de 33 à 99,5 % en poids, de préférence à raison de 50 à 86 % en poids, et tout particulièrement à raison de 55 à 70 % en poids, par rapport au poids total du mélange anhydre.

Le pigment organique peut généralement être présent à raison de 0,49 à 34 % en poids, de préférence à raison de 5 à 30 % en poids, et tout particulièrement à raison de 10 à 15 % en poids, par rapport au poids total du mélange anhydre.

Avantageusement, le pigment organique et le composé silicié (I) sont mis en oeuvre dans le procédé de préparation selon l'invention en un rapport pondéral pigment organique/composé silicié (I) allant de 0,08 à 1, et de préférence allant de 0,3 à 0,8.

L'huile éventuellement présente dans le mélange anhydre peut être choisies parmi les huiles usuelles du domaine cosmétique.
L'huile est avantageusement une huile apolaire, et notamment volatile.
On peut également utiliser comme huile les benzoates d'alcools en C₁₂ à C₁₅ comme ceux vendus sous la dénomination TEGOSOFT TN chez EVONIK GOLDSCHMIDT.

Par « huile apolaire » au sens de la présente invention, on entend une huile dont le paramètre de solubilité à 25°C, δₐ, est égal à 0 (J/cm³)^{½}.
La définition et le calcul des paramètres de solubilité dans l'espace de solubilité tridimensionnel de HANSEN sont décrits dans l'article de C. M. HANSEN : "The three dimension-nal solubility parameters" J. Paint Technol. 39, 105 (1967).
Selon cet espace de Hansen :
- δ_{D} caractérise les forces de dispersion de LONDON issues de la formation de dipôles induits lors des chocs moléculaires ;
- δₚ caractérise les forces d'interactions de DEBYE entre dipôles permanents ainsi que les forces d'interactions de KEESOM entre dipôles induits et dipôles permanents;
- δₕ caractérise les forces d'interactions spécifiques (type liaisons hydrogène, acide/base, donneur/accepteur, etc.) ; et
- δₐ est déterminé par l'équation : δₐ = (δₚ² + δₕ²)^{½}.
Les paramètres δ_{ρ}, δₕ, δ_{D} et δₐ sont exprimés en (J/cm³)^{½}.

Par « huile hydrocarbonée apolaire », on entend une huile formée essentiellement, voire constituée, d'atomes de carbone et d'hydrogène et exempte d'hétéroatomes tel que N, O, Si et P.

L'huile hydrocarbonée apolaire peut également être une huile volatile.

Par « huile volatile », on entend une huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg). Inversement, une huile non volatile a une pression de vapeur inférieure à 0,13 Pa.

Comme exemples d'huiles hydrocarbonées apolaires non volatile, on peut citer les huiles hydrocarbonées comme le squalène, les hydrocarbures linéaires ou ramifiés tels que les huiles de paraffine, de vaseline et de naphtalène, le polybutène, le polyisobutène, le polyisobutène hydrogéné ou partiellement hydrogéné, l'isoeicosane, le squalane, les copolymères décène/butène, les copolymères polybutène/polyisobutène notamment l'Indopol L-14, les polydécènes tel que le PURESYN 10, et leurs mélanges.
En particulier on peut citer les huiles apolaires hydrocarbonées non volatiles de masse moléculaire élevée, également appelées huiles brillantes, la masse moléculaire étant par exemple comprise entre 650 à 10 000 g/mol comme par exemple :
- les polybutylènes tels que L'INDOPOL H-100 (de masse molaire ou MW=965 g/mol), L'INDOPOL H-300 (MW=1340 g/mol), L'INDOPOL H-1500 (MW=2160g/mol) commercialisés ou fabriqués par la société AMOCO,
- les polyisobutylènes hydrogénés tels que le PANALANE H-300 E commercialisés ou fabriqué par la société AMOCO (MW =1340 g/mol), le VISEAL 20000 commercialisé ou fabriqué par la société SYNTEAL (MW=6000 g/mol), le REWOPAL PIB 1000 commercialisé ou fabriqué par la société WITCO (MW=1000 g/mol),
- les polydécènes et les polydécènes hydrogénés tels que le PURESYN 150 (MM=9200 g/mol) commercialisé par la société MOBIL CHEMICALS, et
- leurs mélanges.

Comme huile volatile hydrocarbonée apolaire, on peut citer les huiles volatiles hydrocarbonées ayant de 7 à 16 atomes de carbone et leurs mélanges, de préférence de 8 à 16 et atomes de carbone, notamment les alcanes ramifiés en C₈-C₁₆ comme les iso-alcanes (appelées aussi isoparaffines) en C₈-C₁₆, l'isododécane, l'isodécane, l'isohexadécane, et par exemple les huiles vendues sous les noms commerciaux d'Isopars ou de Permetyls,et leurs mélanges.
Selon un mode de réalisation, les huiles apolaires, volatiles ou non volatils, convenant à l'invention peuvent être choisies parmi le polybutène, le polyisobutène, le polyisobuténe hydrogéné, l'isododécane, l'isohexadécane, et leurs mélanges.

Comme huile volatile hydrocarbonée apolaire, on peut citer les huiles volatiles hydrocarbonées ayant de 7 à 15 atomes de carbone et leurs mélanges, et notamment les alcanes linéaires en C₇-C₁₅.
De façon préférée, les « alcanes linéaires volatils » convenant à l'invention comprennent de 8 à 14 atomes de carbone.
De façon préférée, les « alcanes linéaires volatils » convenant à l'invention comprennent de 9 à 14 atomes de carbone.
De façon préférée, les « alcanes linéaires volatils » convenant à l'invention comprennent de 10 à 14 atomes de carbone.
De façon préférée, les « alcanes linéaires volatils » convenant à l'invention comprennent de 11 à 14 atomes de carbone.

Les alcanes linéaires volatiles pouvant être utilisés dans les compositions selon l'invention peuvent en particulier présenter une pression de vapeur (appelée également pression de vapeur saturante) non nulle, à température ambiante, en particulier une pression de vapeur allant de 0,3 Pa à 6000 Pa.
De façon préférée, les « alcanes linéaires volatils » convenant à l'invention ont une pression de vapeur allant de 0,3 à 2000 Pa, à température ambiante (25°C).
De façon préférée, les « alcanes linéaires volatils » convenant à l'invention ont une pression de vapeur allant de 0,3 à 1000 Pa, à température ambiante (25°C)
De façon plus préférée, les « alcanes linéaires volatils » convenant à l'invention ont une pression de vapeur allant de 0,4 à 600 Pa, à température ambiante (25°C).
De façon préférée, les « alcanes linéaires volatils » convenant à l'invention ont une pression de vapeur allant de 1 à 200 Pa, à température ambiante (25°C).
De façon encore préférée, les « alcanes linéaires volatils » convenant à l'invention ont une pression de vapeur allant de 3 à 60 Pa, à température ambiante (25°C).

A titre d'exemple d'alcanes convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.
A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C11) et de n-tridécane (C13) obtenus aux exemples 1 et 2 de la demande WO2008/155059 de la Société Cognis.

On peut également citer le n-dodécane (C12) et le n-tétradécane (C14) vendus par Sasol respectivement sous les références PARAFOL 12-97 et PARAFOL 14-97, ainsi que leurs mélanges.

L'huile peut être présente dans le mélange anhydre en une teneur allant de 0 à 60 % en poids, par rapport au poids total du mélange anhydre, et de préférence allant de 5 à 50 % en poids.

La phase aqueuse mise en oeuvre dans le procédé de préparation selon l'invention contient un tensioactif tel que défini précédemment.

Le tensioactif peut être choisi parmi les sels d'alkyl(C₈-C₁₆) sulfate , notamment choisi parmi lesdits sels de sodium, de potassium, de magnésium, d'ammonium. Le tensioactif est en particulier choisi parmi les sels d'alkyle(C₁₀-C₁₄) sulfate, notamment choisi parmi lesdits sels de sodium, de potassium, de magnésium, d'ammonium.
Préférentiellement le tensioactif est choisi parmi les sels de lauryle sulfate, notamment choisi parmi lesdits sels de sodium, de potassium, de magnésium, d'ammonium.
Avantageusement, le tensioactif est le laurylsulfate de sodium.

Le tensioactif peut également être choisi parmi les alkyl (C₆-C₁₀) phénol éthoxylé ayant de 5 à 40 motifs d'oxyde d'éthylène. Préférentiellement, le tensioactif est choisi parmi l'octylphénol éthoxylé ayant de 5 à 40 motifs d'oxyde d'éthylène.

On peut utiliser comme tensioactif les composés suivants :
Octylphénol éthoxylé à 5 motifs d'oxyde d'éthylène ; Nom INCI OCTOXYNOL-5 ; comme le Triton X-45 de chez Dow Chemical Company
Octylphénol éthoxylé à 8 motifs d'oxyde d'éthylène ; Nom INCI OCTOXYNOL-8 ; comme le Triton X-114 de chez Dow Chemical Company
Octylphénol éthoxylé à 9 motifs d'oxyde d'éthylène ; Nom INCI OCTOXYNOL-9 ; comme le Triton X-100 de chez Dow Chemical Company
Octylphénol éthoxylé à 13 motifs d'oxyde d'éthylène ; Nom INCI OCTOXYNOL-13 ; comme le Triton X-102 de chez Dow Chemical Company
Octylphénol éthoxylé à 16 motifs d'oxyde d'éthylène ; Nom INCI OCTOXYNOL-16 ; comme le Triton X-165 de chez Dow Chemical Company
Octylphénol éthoxylé à 30 motifs d'oxyde d'éthylène ; Nom INCI OCTOXYNOL-30 ; comme le Triton X-305 de chez Dow Chemical Company
Octylphénol éthoxylé à 40 motifs d'oxyde d'éthylène ; Nom INCI OCTOXYNOL-40 ; comme le Triton X-405 de chez Dow Chemical Company
Préférentiellement, on utilise l'octylphénol éthoxylé ayant de 8 à 16 motifs d'oxyde d'éthylène.

Avantageusement, on utilise l'octylphénol éthoxylé ayant 9 motifs d'oxyde d'éthylène.

Le tensioactif peut être présent dans la composition aqueuse mise en oeuvre dans le procédé de préparation en une teneur allant de 0,1 à 5 % en poids, de préférence allant de 0,2 à 1,5 % en poids, par rapport au poids total de la composition aqueuse.

La composition aqueuse peut comprendre un agent épaississant hydrosoluble non ionique, notamment en une teneur allant de 0,1 % à 10 % en poids, par rapport au poids total de la phase aqueuse.

Parmi les agents épaississants hydrosolubles non ioniques utilisable selon l'invention, on peut citer :
- les épaississants cellulosiques hydrosolubles tels que l'hydroxyéthylcellulose, la méthylcellulose, l'hydroxypropylcellulose ;
- la gomme de guar,
- les gommes de guar non-ioniques comprenant des groupements hydroxyalkyle en C₁-C₆. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120 et JAGUAR HP 105 par la société MEYHALL, ou sous la dénomination GALACTASOL 40H4FD2 par la société AQUALON ;
- les gommes de xanthane, de caroube, de scléroglucane, de gellane,
- la polyvinylpyrrolidone,
- l'alcool polyvinylique.

De préférence, on utilise l'alcool polyvinylique.

Le procédé de préparation du pigment organique enrobé selon l'invention comprend les étapes suivantes :
a) on prépare un mélange anhydre
   (i) d'un pigment organique et (ii) d'un composé silicié de formule (I) :

      [R₁-O]₃Si-R₂ (I)

      dans laquelle R₁ désigne un radical alkyle en C₁-C₄, R₂ désigne un radical alkyle en C₁-C₈ ;
      en l'absence de tétraalkyl(C₁-C₄)orthosilicate ;
      et éventuellement d'une huile ;
   b) ledit mélange est mis en contact avec une composition aqueuse comprenant un tensioactif choisi parmi les sels d'alkyl (C₈-C₁₆) sulfate et les alkyl(C₆-C₁₀)phénol oxyéthyléné comprenant de 5 à 40 motifs d'oxyde d'éthylène ;
   c) récupération du pigment enrobé sous forme de poudre.

On peut laisser agir le mélange final à une température allant de 10 °C à 30 °C, notamment à température ambiante (25 °C). L'agitation dudit mélange peut durer de 5 minutes à 1 heure. Le produit issu du contact du composé silicié (I) avec l'eau se dépose sur le pigment organique et enrobe ce dernier en formant un revêtement de type silice alkylée.
Le milieu réactionnel peut ensuite être filtré ou centrifugé et le filtrat recueilli est lavé, notamment avec de l'eau. Après séchage, on obtient une poudre.
Dans le procédé selon l'invention, le mélange final, le pigment organique, le composé silicié (I) et l'eau, est avantageusement placé sous agitation efficace pour empêcher la formation d'agglomérats et/ou désagréger les agglomérats au fur et à mesure de leur formation. On obtient ainsi après réaction une poudre de pigment organique enrobé. Selon un mode particulier du procédé selon l'invention, ledit mélange final peut être traité aux ultra-sons pour désagréger les agglomérats pouvant se former lors de la réaction.
La taille moyenne des particules de pigment organique enrobé peut aller de 0,1 µm à 500 µm, de préférence de 1 µm à 500 µm, et préférentiellement de 1 µm à 100 µm.
La teneur du produit d'enrobage dans le pigment organique enrobé peut aller de 20 % à 300 % en poids, par rapport au poids de pigment organique (non enrobé), et de préférence de 80 % à 150 % en poids.
Le pigment organique enrobé peut être présent dans la composition cosmétique selon l'invention en une teneur allant de 0,1 % à 99 % en poids, par rapport au poids total de la composition, de préférence de 0,5 % à 80 % en poids, et mieux de 1 % à 60 % en poids.

La composition cosmétique selon l'invention peut se présenter sous la forme d'émulsion eau-dans-huile ou huile-dans-eau, de poudre libre ou compactée, de poudre coulée, de stick solide, de pâte, de lotion organique ou aqueuse.

La composition peut comprendre, en outre, d'autres ingrédients utilisés couramment dans les compositions cosmétiques. De tels ingrédients peuvent être choisis parmi les conservateurs, les parfums, les filtres solaires, les huiles, les cires, les épaississants, les polymères filmogènes, les agents hydratants, les vitamines, les protéines, les céramides, les tensioactifs, les antioxydants, les agents antiradicaux libres, les solvants organiques, l'eau, les pigments minéraux.
Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

L'invention est illustrée plus en détail dans les exemples suivants.

### Exemple 1 :

### a) Pigment organique enrobé :

1,5 g de D&C red No 7 (nom INCI CI15850) a été dispersé dans 10 g de méthyltriéthoxysilane (MTES) pour constituer une phase organique. 0,45 g d'octylphénol oxyéthyléné (9 motifs d'oxyde d'éthylène) (TRITON© X100 de chez Dow Chemical) ont été solubilisés dans 100 g d'eau. Cette phase aqueuse a été émulsionnée avec la phase organique à l'aide d'un rotor stator à 13500 tours/min pendant 10 minutes. En fin d'émulsification, l'émulsion a été maintenue sous agitation magnétique, et 0,17 g de HCl 0,1 M a été additionné. Après 10 minutes, on a ajouté de 0,21 g d'une solution aqueuse d'ammoniaque à 20 %. Le mélange a été laissé sous agitation pendant 24 heures, puis centrifugé pendant 30 minutes à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenue : le pigment présente une fluorescence exaltée.

### Exemple 2 :

0,56 g de D&C red No 7 (nom INCI CI15850) a été dispersé dans 10 g de méthyltriéthoxysilane pour constituer une phase organique. 0,45 g d' octylphénol oxyéthyléné (9 motifs d'oxyde d'éthylène) (TRITON© X100 de chez Dow Chemical) ont été solubilisés dans 100 g d'eau. Cette phase aqueuse a été émulsionnée avec la phase organique à l'aide d'un rotor stator à 13500 tours/min pendant 10 minutes. En fin d'émulsification, l'émulsion a été maintenue sous agitation magnétique, et 0,17g de HCl 0,1M a été additionné. Après 10 minutes, ajout de 0,21 g d'une solution aqueuse d'ammoniaque à 20 % en poids. Le mélange a été laissé sous agitation pendant 24 heures, puis centrifugé 30 min à 4000 tours/min, puis lavé à l'eau, puis centrifugé à nouveau. La poudre obtenue a été récupérée sur papier filtre puis séchée à l'air.

On a évalué sous lampe UV 365 nm la fluorescence du pigment enrobé obtenu en comparaison de celle du pigment non enrobé : le pigment enrobé présente une fluorescence exaltée.

### Exemple 3 :

1,5 g de D&C red No 7 (nom INCI CI15850) a été dispersé dans 5 g de benzoate d'alcool en C12-C15 (TEGOSOFT TN de chez EVONIK GOLDSCHMIDT) et 10 g de méthyltriéthoxysilane pour constituer une phase organique. 0,42 g d' octylphénol oxyéthyléné (9 motifs d'oxyde d'éthylène) (TRITON© X100 de chez Dow Chemical) ont été solubilisés dans 100 g d'eau. Cette phase aqueuse a été émulsionnée avec la phase organique à l'aide d'un rotor stator à 13500tours/min pendant 10 minutes. En fin d'émulsification, l'émulsion a été maintenue sous agitation magnétique, et 0,17g de HCl 0,1M a été additionné. Après 10 minutes, ajout de 0,21g d'une solution aqueuse d'ammonique à 20 % en poids . Le mélange a été laissé sous agitation pendant 24 heures, puis centrifugé 30 min à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenu en comparaison de celle du pigment non enrobé : le pigment enrobé présente une fluorescence exaltée.

### Exemple 4 :

2,52 g de D&C red No 7 (nom INCI CI15850) ont été dispersés dans 23,7g de méthyltriéthoxysilane et 0,84 g d'isododécane pour constituer une phase organique. 1,68g de lauryléthersulfate de sodium a été solubilisé dans 200 g d'eau contenant 5 % en poids de PVA hydrolysé à 98 % (Mw=13000-25000) (CELVOL ® 305 de chez CELANESE CHEMICALS). Les deux phases ont été émulsionnées avec un sonicateur puis le mélange a été laissé sous agitation pendant 5 heures. La poudre obtenue a été récupéé par filtration sur papier.

On a évalué sous lampe UV 365 nm la fluorescence du pigment enrobé obtenu en comparaison de celle du pigment non enrobé : le pigment enrobé présente une fluorescence exaltée.

### Exemple 5 (hors invention) :

0,56 g de D&C red No 7 (nom INCI CI15850) a été dispersé dans 1 g de méthyltriéthoxysilane et 9 g de tétraéthylorthosilicate pour constituer une phase organique. 0,45 g d' octylphénol oxyéthyléné (9 motifs d'oxyde d'éthylène) (TRITON© X100 de chez Dow Chemical) ont été solubilisés dans 100 g d'eau. Cette phase aqueuse a été émulsionnée avec la phase organique à l'aide d'un rotor stator à 13500 tours/min pendant 10 min. En fin d'émulsification, l'émulsion a été maintenue sous agitation magnétique, et 0,17g de HCl 0,1M a été additionné. Après 10 minutes, ajout de 0,21g d'une solution de NH₃ à 20% . Le mélange a été laissé sous agitation pendant 24 heures, puis centrifugé 30 min à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenue : la fluorescence n'est pas exaltée en comparaison de celle du pigment non enrobé.

### Exemple 6 (hors invention) :

0,56 g de D&C red No 7 (nom INCI CI15850) a été dispersé dans 9g de méthyltriéthoxysilane et 1g de tétraéthylorthosilicate afin de constituer une phase organique. 0,45g d'octylphénol oxyéthyléné (9 motifs d'oxyde d'éthylène) (TRITON© X100 de chez Dow Chemical) ont été solubilisés dans 100 g d'eau. Cette phase aqueuse a été émulsionnée avec la phase organique à l'aide d'un rotor stator à 13500 tours/min pendant 10 min. En fin d'émulsification, l'émulsion est maintenue sous agitation magnétique, et 0,17g de HCl 0,1M est additionné. Après 10 minutes, ajout de 0,21g d'une solution de NH₃ à 20% . Le mélange a été laissé sous agitation pendant 24 heures, puis centrifugé 30 min à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenue : la fluorescence n'est pas exaltée en comparaison de celle du pigment non enrobé.

### Exemple 7 (hors invention) :

0,56 g de D&C red No 7 (nom INCI CI15850) a été dispersé dans 10 g de méthyltriéthoxysilane pour constituer une phase organique. 0,45 g de condensat d'oxyde d'ethylene et d'oxyde de propylene et d'oxyde d'ethylene (75 OE/30 OP/75 OE) (PLURACARE/PLURONIC F 68 PRILL de chez BASF) ont été solubilisés dans 100 g d'eau. Cette phase aqueuse a été émulsionnée avec la phase organique à l'aide d'un rotor stator à 13500 tours/min pendant 10 min. En fin d'émulsification, l'émulsion a été maintenue sous agitation magnétique, et 0,17g de HCl 0,1M a été additionné. Après 10 minutes, ajout de 0,21g d'une solution de NH₃ à 20% . Laisser sous agitation pendant 24heures. Le mélange a été laissé sous agitation pendant 24 heures, puis centrifugé 30 min à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenue : la fluorescence n'est pas exaltée en comparaison de celle du pigment non enrobé.

### Exemple 8 (hors invention) :

1,5 g de D&C red No 7 (nom INCI CI15850) a été dispersé dans 10 g de méthyltriéthoxysilane pour constituer une phase organique. 0,45g de diester formé par réaction d'octyldodécanol et de PPG-3 Myristyl Ether avec l'acide dilinoléique (dimère) (Nom INCI: OCTYLDODECYL/PPG-3 MYRISTYL ETHER DIMER DILINOLEATE) (LIQUIWAX POLYEFA OR de chez Arch personnal care) ont été solubilisés dans 100 g d'eau. Cette phase aqueuse a été émulsionnée avec la phase organique à l'aide d'un rotor stator à 13500 tours/min pendant 10 minutes. En fin d'émulsification, l'émulsion a été maintenue sous agitation magnétique, et 0,17 g de HCl 0,1M a été additionné. Après 10 minutes, ajout de 0,21g d'une solution de NH₃ à 20% . Le mélange a été laissé sous agitation pendant 24 heures, puis centrifugé 30 min à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenue : la fluorescence n'est pas exaltée en comparaison de celle du pigment non enrobé.

### Exemple 9 (hors invention) :

0,56 g de D&C red No 7 (nom INCI CI15850) a été dispersé dans 10 g de méthyltriéthoxysilane afin de constituer une phase organique. 0,45g de condensat d'oxyde d'ethylene et d'oxyde de propylene et d'oxyde d'ethylene (17 OE/60 OP/17 OE) (PLURONIC P 103 de chez BASF) ont été solubilisés dans 100 g d'eau. Cette phase aqueuse a été émulsionnée avec la phase organique à l'aide d'un rotor stator à 13500 tours/min pendant 10 minutes. En fin d'émulsification, l'émulsion a été maintenue sous agitation magnétique, et 0,17g de HCl 0,1M a été additionné. Après 10 minutes, ajout de 0,21g d'une solution de NH₃ à 20% . Le mélange a été laissé sous agitation pendant 24 heures, puis centrifugé 30 min à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenue : la fluorescence n'est pas exaltée en comparaison de celle du pigment non enrobé.

### Exemple 10 :

On a préparé un vernis à ongles ayant la composition suivante :

| | |
|---|---|
| - Nitrocellulose | 10 g |
| - Plastifiants et résine | 15 g |
| - Agent rhéologique | 1,5 g |
| - Pigment organique enrobé de l'exemple 1 | 10 g |
| - Acétate d'éthyle, acétate de butyle qsp | 100 g |

Après application de la composition sur les ongles, on a obtenu un film de maquillage de couleur rouge vif.

Une composition similaire est préparée avec le pigment organique de l'exemple 2 ou de l'exemple 3 ou de l'exemple 4.

1,5 g de D&C red No 7 (nom INCI CI15850) a été dispersé dans 10 g de méthyltriéthoxysilane pour constituer une phase organique. 0,45g de diester formé par réaction d'octyldodécanol et de PPG-3 Myristyl Ether avec l'acide dilinoléique (dimère) (Nom INCI : OCTYLDODECYL/PPG-3 MYRISTYL ETHER DIMER DILINOLEATE) (LIQUIWAX POLYEFA OR de chez Arch personnal care) ont été solubilisés dans 100 g d'eau. Cette phase aqueuse a été émulsionnée avec la phase organique à l'aide d'un rotor stator à 13500 tours/min pendant 10 minutes. En fin d'émulsification, l'émulsion a été maintenue sous agitation magnétique, et 0,17 g de HCl 0,1M a été additionné. Après 10 minutes, ajout de 0,21g d'une solution de NH₃ à 20% . Le mélange a été laissé sous agitation pendant 24 heures, puis centrifugé 30 min à 4000 tours/min, puis lavé à l'eau, puis centrifugé à nouveau. La poudre obtenue a été récupérée sur papier filtre puis séchée à l'air.

On a évalué sous lampe UV 365 nm la fluorescence du pigment enrobé obtenue : la fluorescence n'est pas exaltée en comparaison de celle du pigment non enrobé.

### Exemple 9 (hors invention) :

0,56 g de D&C red No 7 (nom INCI CI15850) a été dispersé dans 10 g de méthyltriéthoxysilane afin de constituer une phase organique. 0,45g de condensat d'oxyde d'ethylene et d'oxyde de propylene et d'oxyde d'ethylene (17 OE/60 OP/17 OE) (PLURONIC P 103 de chez BASF) ont été solubilisés dans 100 g d'eau. Cette phase aqueuse a été émulsionnée avec la phase organique à l'aide d'un rotor stator à 13500 tours/min pendant 10 minutes. En fin d'émulsification, l'émulsion a été maintenue sous agitation magnétique, et 0,17g de HCl 0,1M a été additionné. Après 10 minutes, ajout de 0,21g d'une solution de NH₃ à 20% . Le mélange a été laissé sous agitation pendant 24 heures, puis centrifugé 30 min à 4000 tours/min, puis lavé à l'eau , puis centrifugé à nouveau. La poudre obtenue a été récupérée sur papier filtre puis séchée à l'air.

On a évalué sous lampe UV 365 nm la fluorescence du pigment enrobé obtenue : la fluorescence n'est pas exaltée en comparaison de celle du pigment non enrobé.

### Exemple 10 :

On a préparé un vernis à ongles ayant la composition suivante :

| | |
|---|---|
| - Nitrocellulose | 1 0 g |
| - Plastifiants et résine | 15 g |
| - Agent rhéologique | 1,5 g |
| - Pigment organique enrobé de l'exemple 1 | 10 g |
| - Acétate d'éthyle, acétate de butyle qsp | 100 g |

## Revendications

1. Procédé de préparation d'un pigment organique enrobé comprenant les étapes suivantes :
a) on prépare un mélange anhydre
(i) d'un pigment organique et (ii) d'un composé silicié de formule (I) :
[R₁-O]₃Si-R₂ (I)
dans laquelle R₁ désigne un radical alkyle en C₂-C₃ , R₂ désigne un radical alkyle en en C₁-C₄;
en l'absence de tétraalkyl(C₁-C₄)orthosilicate ;
et éventuellement (iii) d'une huile ;
b) ledit mélange est mis en contact avec une composition aqueuse comprenant un tensioactif choisi parmi les sels d'alkyl (C₈-C₁₆) sulfate et les alkyl(C₆-C₁₀)phénol oxyéthyléné comprenant de 5 à 40 motifs d'oxyde d'éthylène ;
c) récupération du pigment enrobé sous forme de poudre .

2. Procédé selon la revendication 1, **caractérisé par le fait que** le pigment organique est choisi parmi :
- le carmin de cochenille,
- les pigments organiques de colorants azoïques, anthraquinoniques, indigoïdes, xanthéniques, pyréniques, quinoliniques, de triphénylméthane, de fluorane ;
- les laques organiques ou sels insolubles de sodium, de potassium, de calcium, de baryum, d'aluminium, de zirconium, de strontium, de titane, de colorants acides azoïques, anthraquinoniques, indigoïdes, xanthéniques, pyréniques, quinoliniques, de triphénylméthane, de fluorane ;
- le D&C red No 7 ;
- les laques de curcumine, de riboflavine, d'azorubine, d'amarante, de chlorophylle, de caroténoïdes, d'anthocyanes, de sorgho, d'acide carminique.

3. Procédé selon la revendication 1 ou 2, **caractérisé par le fait que** le pigment organique est le D&C red No 7 .

4. Procédé selon l'une quelconque des revendications précédentes, **caractérisé par le fait que** le composé silicié (I) est le méthyltriéthoxysilane.

5. Procédé selon l'une quelconque des revendications précédentes, **caractérisé par le fait que** le pigment organique est présent dans le mélange anhydre en une teneur allant de 0,49 à 34 % en poids, par rapport au poids total du mélange anhydre.

6. Procédé selon l'une quelconque des revendications précédentes, **caractérisé par le fait que** le composé silicié (I) est présent dans le mélange anhydre en une teneur allant de de 33 à 99,5 % en poids, par rapport au poids total du mélange anhydre.

7. Procédé selon l'une quelconque des revendications précédentes, **caractérisé par le fait que** le pigment organique et le composé silicié (I) sont mis en oeuvre en un rapport pondéral pigment organique/composé silicié (I) allant de 0,08 à 1.

8. Procédé selon l'une quelconque des revendications précédentes, **caractérisé par le fait que** l'huile éventuellement présente est une huile apolaire.

9. Procédé selon l'une quelconque des revendications précédentes, **caractérisé par le fait que** le tensioactif est choisi parmi le laurylsulfate de sodium et l'octylphénol éthoxylé ayant de 5 à 40 motifs d'oxyde d'éthylène.

10. Procédé selon l'une quelconque des revendications précédentes, **caractérisé par le fait que** le tensioactif est présent dans la composition aqueuse en une teneur allant de 0,1 à 5 % en poids, de préférence allant de 0,2 à 1,5 % en poids, par rapport au poids total de la composition aqueuse.

## Patentansprüche

1. Verfahren zur Herstellung eines beschichteten organischen Pigments, das die folgenden Schritte umfasst:
a) Herstellen einer wasserfreien Mischung
(i) eines organischen Pigments und (ii) einer Siliciumverbindung der Formel (I) :
[R₁-O]₃Si-R₂ (I)
in der R₁ für einen C₂-C₃-Alkylrest steht und R₂ für einen C₁-C₄-Alkylrest steht;
in Abwesenheit von Tetra(C₁-C₄)alkylorthosilicat;
und gegebenenfalls (iii) eines Öls;
b) Inkontaktbringen der Mischung mit einer wässrigen Zusammensetzung, die ein aus (C₈-C₁₆) Alkylsulfatsalzen und oxyethylenierten (C₆-C₁₀)-Alkylphenolen mit 5 bis 40 Ethylenoxid-Einheiten ausgewähltes Tensid umfasst;
c) Gewinnen des beschichteten Pigments in Pulverform.

2. Verfahren nach Anspruch 1, **dadurch gekennzeichnet, dass** das organische Pigment aus:
- Cochenill-Karmin,
- organischen Pigmenten von Azo-, Anthrachinon-, Indigoid-, Xanthen-, Pyren-, Chinolin-, Triphenylmethan- und Fluoran-Farbstoffen;
- organischen Lacken oder unlöslichen Natrium-, Kalium-, Calcium-, Barium-, Aluminium-, Zirconium-, Strontium- und Titansalzen von sauren Azo-, Anthrachinon-, Indigoid-, Xanthen-, Pyren-, Chinolin-, Triphenylmethan- und Fluoran-Farbstoffen;
- D&C Red No. 7;
- Curcumin-, Riboflavin-, Azorubin-, Amarant-, Chlorophyll-, Carotinoid-, Anthocyan-, Sorgho- und Carminsäurelacken
ausgewählt wird.

3. Verfahren nach Anspruch 1 oder 2, **dadurch gekennzeichnet, dass** es sich bei dem organischen Pigment um D&C Red No. 7 handelt.

4. Verfahren nach einem der vorhergehenden Ansprüche, **dadurch gekennzeichnet, dass** es sich bei der Siliciumverbindung (I) um Methyltriethoxysilan handelt.

5. Verfahren nach einem der vorhergehenden Ansprüche, **dadurch gekennzeichnet, dass** das organische Pigment in der wasserfreien Mischung in einem Gehalt im Bereich von 0,49 bis 34 Gew.-%, bezogen auf das Gesamtgewicht der wasserfreien Mischung, vorliegt.

6. Verfahren nach einem der vorhergehenden Ansprüche, **dadurch gekennzeichnet, dass** die Siliciumverbindung (I) in der wasserfreien Mischung in einem Gehalt im Bereich von 33 bis 99,5 Gew.-%, bezogen auf das Gesamtgewicht der wasserfreien Mischung, vorliegt.

7. Verfahren nach einem der vorhergehenden Ansprüche, **dadurch gekennzeichnet, dass** das organische Pigment und die Siliciumverbindung (I) in einem Gewichtsverhältnis von organischem Pigment zu Siliciumverbindung (I) im Bereich von 0,08 bis 1 eingesetzt werden.

8. Verfahren nach einem der vorhergehenden Ansprüche, **dadurch gekennzeichnet, dass** es sich bei dem gegebenenfalls vorliegenden Öl um ein unpolares Öl handelt.

9. Verfahren nach einem der vorhergehenden Ansprüche, **dadurch gekennzeichnet, dass** das Tensid aus Natriumlaurylsulfat und ethoxyliertem Octylphenol mit 5 bis 40 Ethylenoxid-Einheiten ausgewählt wird.

10. Verfahren nach einem der vorhergehenden Ansprüche, **dadurch gekennzeichnet, dass** das Tensid in der wässrigen Zusammensetzung in einem Gehalt im Bereich von 0,1 bis 5 Gew.-%, vorzugsweise im Bereich von 0,2 bis 1,5 Gew.-%, bezogen auf das Gesamtgewicht der wässrigen Zusammensetzung, vorliegt.

## Claims

1. Process for preparing a coated organic pigment, comprising the following steps:
a) an anhydrous mixture
(i) of an organic pigment and (ii) of a siliceous compound of formula (I):
[R₁-O]₃Si-R₂ (I)
in which R₁ denotes a C₂-C₃ alkyl radical and R₂ denotes a C₁-C₄ alkyl radical;
is prepared, in the absence of tetra(C₁-C₄)alkyl orthosilicate;
and optionally (iii) of an oil;
b) said mixture is placed in contact with an aqueous composition comprising a surfactant chosen from (C₈-C₁₆) alkyl sulfate salts and oxyethylenated (C₆-C₁₀) alkylphenols comprising from 5 to 40 ethylene oxide units;
c) the coated pigment in powder form is recovered.

2. Process according to Claim 1, **characterized in that** the organic pigment is chosen from:
- cochineal carmine,
- organic pigments of azo dyes, anthraquinone dyes, indigoid dyes, xanthene dyes, pyrene dyes, quinoline dyes, triphenylmethane dyes or fluoran dyes;
- organic lakes or insoluble sodium, potassium, calcium, barium, aluminum, zirconium, strontium or titanium salts of acidic azo, anthraquinone, indigoid, xanthene, pyrene, quinoline, triphenylmethane or fluoran dyes;
- D&C Red No. 7;
- curcumin, riboflavin, azorubin, amaranth, chlorophyll, carotenoid, anthocyan, sorghum or carminic acid lakes.

3. Process according to Claim 1 or 2, **characterized in that** the organic pigment is D&C Red No. 7.

4. Process according to any one of the preceding claims, **characterized in that** the siliceous compound (I) is methyltriethoxysilane.

5. Process according to any one of the preceding claims, **characterized in that** the organic pigment is present in the anhydrous mixture in a content ranging from 0.49% to 34% by weight, relative to the total weight of the anhydrous mixture.

6. Process according to any one of the preceding claims, **characterized in that** the siliceous compound (I) is present in the anhydrous mixture in a content ranging from 33% to 99.5% by weight, relative to the total weight of the anhydrous mixture.

7. Process according to any one of the preceding claims, **characterized in that** the organic pigment and the siliceous compound (I) are used in an organic pigment/siliceous compound (I) weight ratio ranging from 0.08 to 1.

8. Process according to any one of the preceding claims, **characterized in that** the oil optionally present is an apolar oil.

9. Process according to any one of the preceding claims, **characterized in that** the surfactant is chosen from sodium lauryl sulfate and ethoxylated octylphenol containing from 5 to 40 ethylene oxide units.

10. Process according to any one of the preceding claims, **characterized in that** the surfactant is present in the aqueous composition in a content ranging from 0.1% to 5% by weight and preferably ranging from 0.2% to 1.5% by weight relative to the total weight of the aqueous composition.
